# EUROPEAN PATENT APPLICATION

(11) **EP 1 702 564 A1**
(43) Date of publication of application: **20.09.2006**
(21) Application number: 05380049.6
(22) Date of filing: 18.03.2005
(51) Int. Cl.: A61B 5/15

(54) **Lancet activating device**

(71) Applicant: Futumeds SDN. BHD, Puchong 47100 Selangor (MY)
(72) Inventor: Yeoh Seng Khoon, Puchong 47100, Selangor (MY); Ong Eng Kang, Puchong 47100, Selangor (MY); Wong Chee Hock, Puchong 47100, Selangor (MY)
(74) Representative: De Pablos Riba, Julio

(57) **Abstract**

A lancet activating device comprising a housing (3) which houses a reciprocating springloaded plunger (17), serving as a trigger to activate a lancet (26); cavity (5,6) for placements of a lancet (26) and a finger (23); apertures (9, 7) for the insertion of said lancet and said finger; leaf-spring (10) to position a finger (23). An unactivated fresh lancet (26) is inserted into the cavity (6) of the lancet activating device and the patient's finger (23) is placed into the cavity (5), whereby the fmger positioning will be determined by the leaf-spring (10). At a press of the top mounted reciprocating plunger (17), the lancet (26) is activated to puncture the finger (23) and the patient proceeds to withdraw his finger and collection of the micro blood sample can proceed. Thereafter a fresh lancet (26) can be inserted into the cavity (6) of the lancet activating device from the lancet insertion aperture (9). The fresh lancet (26) forces the used lancet (27) out of the lancet activating device via the lancet disposition aperture (8) where it will be transited to an attached container (2) via an access opening (16) located at the top of the container (2). Throughout the procedure, the users of said lancet activating device do not need to come into contact with the patient.

## Description

### Field of Invention

This invention relates to medical equipment, more specifically, a lancet activating device.

### Background of Invention

Lancets are now commonly used implements for obtaining micro blood samples which are required for a myriad of medical screening and testing procedures. While the use of lancets greatly assists and eases the process of blood sample extraction, the health worker will need to hold the patient's finger for the blood sample extraction and is in direct contact with the patient during extraction. Therefore, the inherent risk of cross-contamination or infection between the patient and health worker cannot be avoided.

Current code of practice during blood sampling requires medical practitioners or support staff to use a fresh pair of examination gloves with each new patient or test subject. In practice however, this is not always possible due to associated costs and occasionally lack of training.

### Object Of the Invention

As the stakes at risk include the loss of lives and potentially large lawsuits for medical malpractice, it is the object of this invention to provide a lancet activating device which is capable of avoiding direct human contact between the user and patient and reducing the risk of cross contamination or infection.

### Summary of the Invention

The present invention is directed to a device for activating a lancet that satisfies the need of avoiding direct human contact between the user and patient. The basic embodiment of the present invention comprises of a housing with a cavity, at least one aperture on said housing to allow means of lancet access into the said cavity, at least one aperture on said housing to allow means of a finger access into the cavity, a means provided within said cavity for positioning the finger to receive the activation of a lancet and an activating means to activate a lancet. When a lancet is inserted into said lancet access means and a finger inserted into the finger access means, the finger is positioned by the means for positioning a finger to receive the activation of a lancet, the activating means is activated to trigger said lancet to prick the finger.

For the basic embodiment of the lancet activating device, the lancet access means is preferably perpendicular to said finger access means and the activating means is preferably a reciprocating plunger. The means for positioning finger is preferably a leaf spring detachably mounted onto the housing and can act as a rest pad for the finger. A stopper is provided in the housing to limit the positioning of the finger. A pair of grooves is also provided on the lancet access means to guide the access of a lancet.

In the basic embodiment of the lancet activating device, the housing is preferably provided with two apertures for lancet access means such that the first aperture is longer than the second aperture. A means of determining the positioning of a lancet is also provided on the housing.

A container can be detachably attached to the basic embodiment of the lancet activating device or it can be permanently attached to the lancet. It is preferred that said container is provided with an opening on the top of the container positioned to receive used lancets. Said opening can be provided with a lid pivotally mounted to close said opening.

### Brief Description of the Drawings

FIG. 1A and 1B depict the isometric view of the preferred embodiment of the present invention.
FIG. 2A and 2B depict the cross-sectional of the isometric view of the preferred activator (1) of present invention.
FIG. 3 depicts the cross-sectional view of the preparation of the preferred embodiment of the present invention for the lancing process.
FIG. 4 depicts the cross-sectional view of the preparation of lancing process.
FIG. 5 depicts the cross-sectional view of the lancing process.
FIG. 6A and 6B depict the cross-sectional view of preferred embodiment of the present invention after the activation.
FIG. 7A and 7B depict the cross-sectional view for the replacement of a fresh lancet and disposition of the used lancet.
FIG. 8 depicts the cross-sectional view of the replacement of the box-container.

### Detailed Description of the Invention

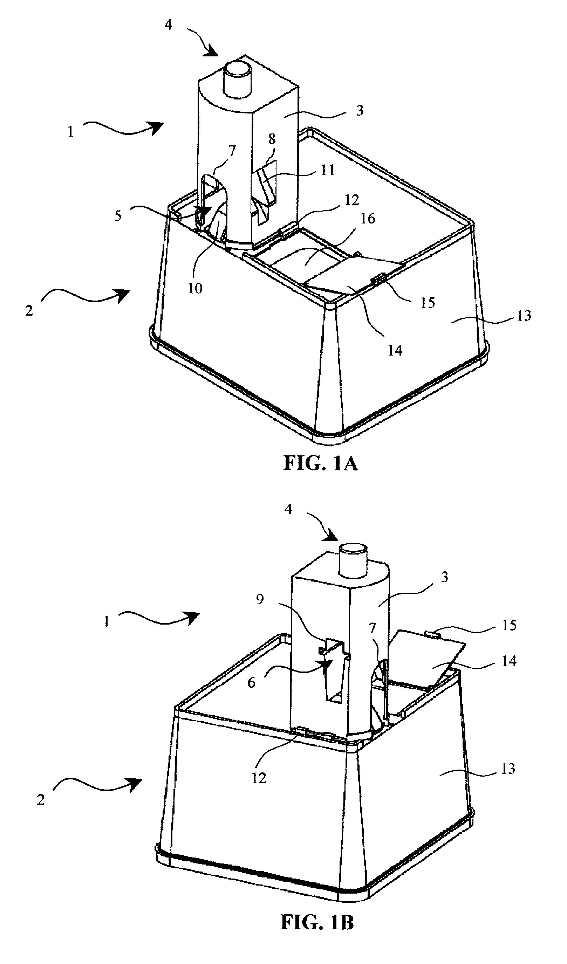

FIG. 1A and 1B illustrate different isometric views of the preferred embodiment of present invention. The preferred lancet activating device comprises an activator (1) and a container (2). The activator (1) comprises a housing (3) with a cavity (5, 6), a lancet activating means (4), a lancet insertion aperture (9) on one end of a lancet placement cavity (6), a lancet disposition aperture (8) on the other end of lancet placement cavity (6), a finger insertion aperture (7), a finger placement cavity (5), and a leaf-spring (10) permanently or removably mounted on the housing (3). The lancet insertion aperture (9) is longer than the lancet disposition aperture (8). Both the lancet insertion aperture (9) and the lancet disposition aperture (8) are not placed directly horizontal to one another and the lancet insertion aperture (9) is located higher than the lancet disposition aperture (8). The container (2) comprises a receptacle (13), an opening (16) and a pivotally mounted lid (14) for the opening.

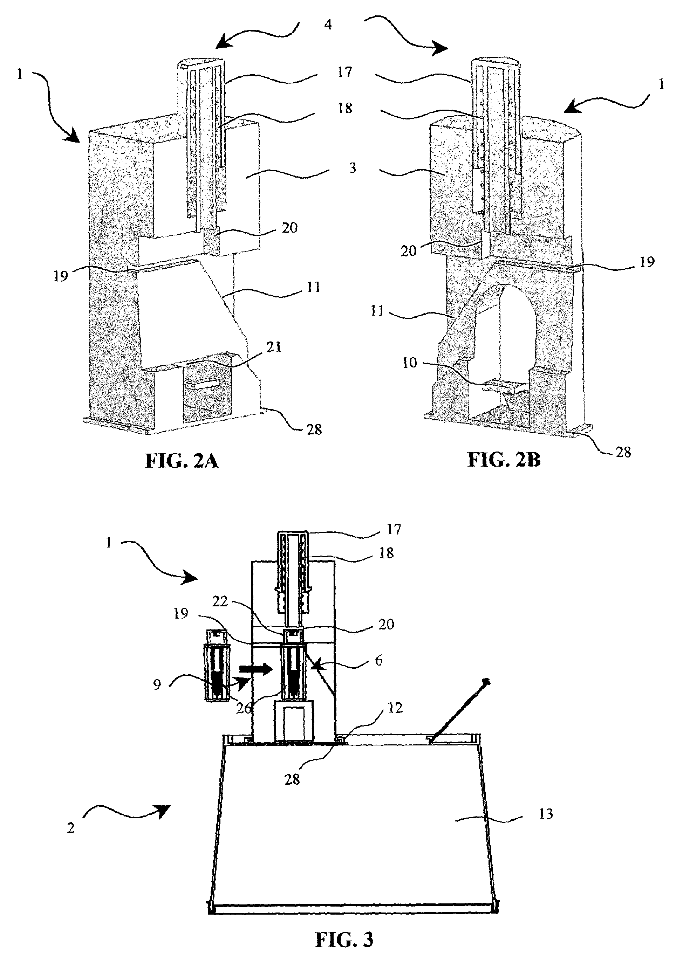

FIG. 2A and 2B illustrate cross-section of the isometric view of the activator (1) of the preferred embodiment of present invention. A pair of grooves (19) is provided on the housing (3) that extends from the lancet insertion aperture (9) into the housing (3). The grooves guide the insertion of the lancet into the activator (1). On the other end of the groove (19), the upper end of the groove (19) is provided with means for preventing an unactivated lancet from further lateral movement (20) thus determining the positioning of said lancet and the lower end of the groove (19) is joined with a sliding slope (11). The lancet activating means (4) comprises a reciprocating plunger (17) and spring (18).

FIG. 3 illustrates the cross-section of the preparation stage of the preferred embodiment of the lancet activating device. The flange (28) on the activator (1) is slid into the notches (12) on the box container (2) for detachably attaching the activator (1) with the container (2). The modular nature of the preferred embodiment of the lancet activating device is optional. The said lancet activating device can be formed with the activator (1) and container (2) as a single item. A fresh lancet (26) is inserted into the lancet placement cavity (6) through the lancet insertion aperture (9) and is glided along the groove (19) until a means of preventing an unactivated lancet from further lateral movement (20) blocks the slider (22) of the lancet (26) from gliding further and the lancet (26) is positioned in place. This completes the preparation stage of the preferred embodiment of the invention and the lancet activating device is ready for lancing.

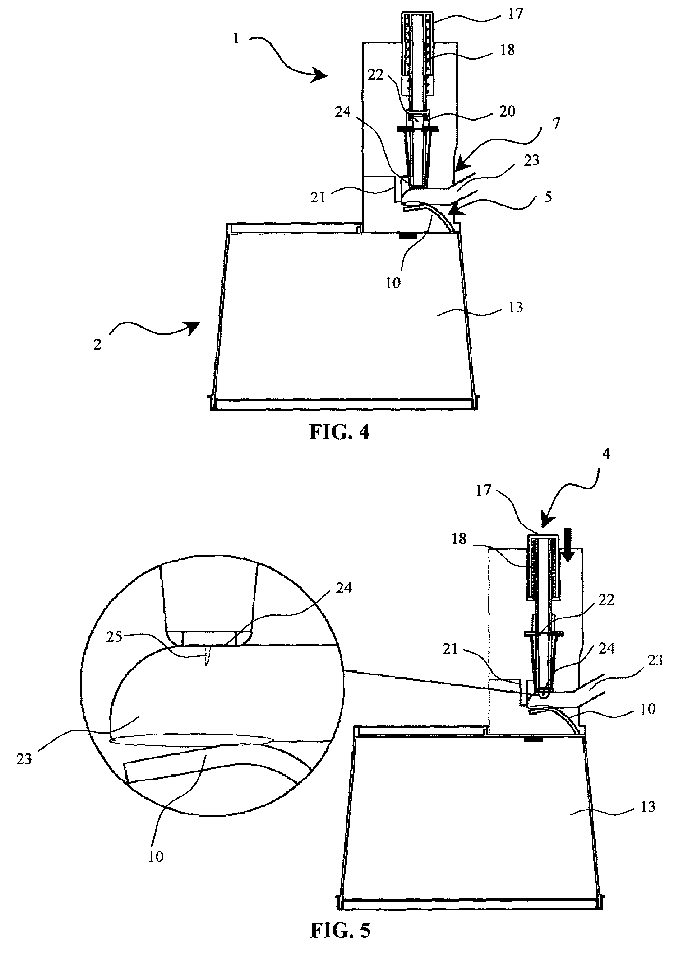

As in FIG. 4, the patient's finger (23) is inserted into the finger placement cavity (5) through the finger insertion aperture (7), thereby compressing the leaf-spring (10). A finger stopper (21) is provided on the housing (3) to allow a pre-determined length of patient's fmger (23) to be inserted into the finger placement cavity (5) so that a more accurate positioning of the finger (23) is achieved. The compressed leaf-spring (10) forces the finger (23) to contact with the lancet (26). The said leaf-spring can also act as a finger rest pad. Additional leaf-spring or springs (not shown) can be mounted on the side of the finger placement cavity (5) to minimise the sideway movement of the patient's finger (23) and or to guide the finger (23) to a position where said finger (23) is positioned at the optimal or preferred point of contact with the lancet (26).

FIG. 5 shows the activation process of the preferred embodiment of present invention. The reciprocating plunger (17) of the lancet activating means (4) is depressed. This action causes the spring (18) to be compressed and energy is stored within the spring. When pressure is applied to the plunger (17), the other end of the plunger that is in contact with the slider (22) will force the slider (22) down and trigger the activation of the lancet (26). The lancet needle (25) will be protmded through the orifice located at the contact surface (24) of the lancet (26) and prick the patient's finger (23). The needle (25) will be retracted after the activation. There is no direct intervention requiring human contact between the medical practitioner and the patient during the lancing process.

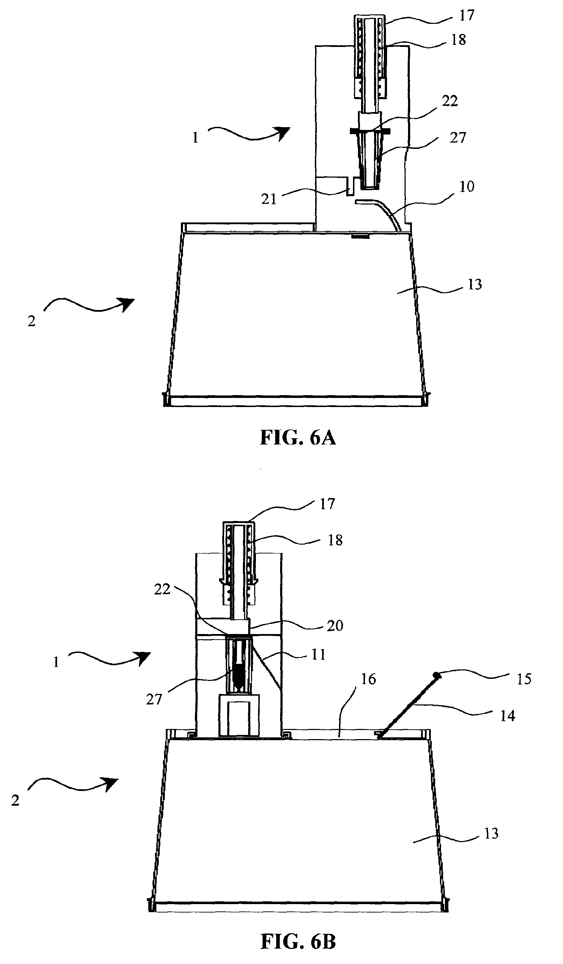

FIG. 6A and 6B depict the cross-sectional view of preferred embodiment of the present invention after the activation. The energy that stored within the spring (18) will retract the reciprocating plunger (17) to its original position when the force imposed to it has been released. The slider (22) of the lancet (26) will be locked into the lancet (26) after the activation. With the absence of the slider (22), the used lancet (27) is no longer blocked by the means for preventing an unactivated lancet from further lateral movement (20).

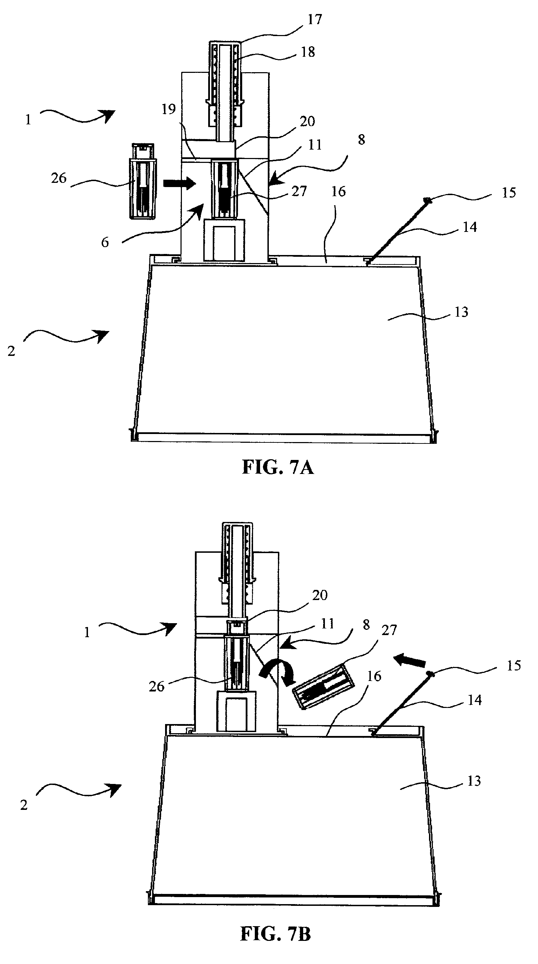

FIG. 7A and 7B are the cross-sectional view for the replacement of a fresh lancet. The insertion process of a fresh lancet (26) into the lancet placement cavity (6) is as earlier described. The insertion of the fresh lancet (26) forces the used lancet (27) to glide down the sliding slope (11). The slider (22) of the fresh lancet (26) will come in contact with the means for preventing an unactivated lancet from further lateral movement (20) and is positioned in place. The used lancet (27) exits the activator (1) through the lancet disposition aperture (8) and drops into the container (2) through the opening (16). The opening (16) is strategically positioned to receive the used lancet (27). Another round of the lancing process can be carried out with the lancet activating device.

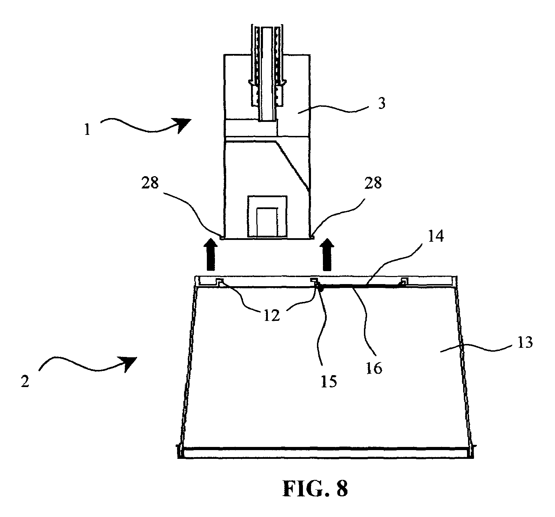

After each lancing process, a fresh lancet (26) is fed into the lancet activating device for a new lancing process. When the container (2) is full of used lancets (27), the lid (14) that is pivotally mounted on the container (2) is used to close the opening (16). The latch (15) on the edge of the lid (14) will lock the lid in place to prevent spillage of the used lancets and attached wastes in the receptacle (13). The container (2) is detached from the activator (I) and disposed of, and a fresh empty container is detachably attached with the activator (1) by sliding its flange (28) into the notches (12) as illustrated in FIG. 8. Alternatively, the entire lancet activating device can be disposed of, especially in the case where the lancet activating device comprises a single non-modular item. The lancet activating device can be constructed from any constructible material, it is preferably constructed of and not limited to polymeric material.

The activator (1) can also be used without a container (2) attached. The activator forms the basic and primary embodiment of the present invention. With the absence of a container (2) attached, once the used lancet (27) exits from the activator, the medical practitioner will have to manually discard the used lancet (27) into a proper dispensing bin and/ or position a proper dispensing means below the activator to catch the used lancet (27).

The second embodiment of the invention is not illustrated. The activator (1) of the second embodiment is similar to that of the preferred embodiment except that the activator (1) of the second embodiment is not provided with a plunger (17) as a lancet activating means. Placement of a fresh lancet (26) is similar as that of the preferred embodiment. In this embodiment, the lancet activating means is provided by the patient's finger (23) whereby the patient is required to manually move the finger upwards to depress the lancet (26). The slider (22) of the lancet (26) will be forced against the housing or any protrusion from the housing to cause the activation of the lancet, thereby activating the lancet. The replacement of the used lancet is performed in the similar manner as that of the preferred embodiment and similarly, the activator (1) can be used on its own without a container (2) attached.

The third embodiment of the invention is also not illustrated. The activator (1) of the second embodiment is similar to that of the preferred embodiment except that the activator of the third embodiment is not provided with a sliding slope (11) and a lancet disposition aperture (8). Placement of a fresh lancet (26) is as described for the preferred embodiment. The activation of the fresh lancet is as described for the preferred embodiment. The removal of the used lancet is done via the lancet insertion aperture (9) by tipping or turning of the activator (1).

The fourth embodiment of the invention is also not illustrated. The activator (1) of the fourth embodiment is similar to that of the preferred embodiment except that the lancet insertion aperture (9) and the lancet disposition aperture (8) are horizontal to one another. The fourth embodiment is also different by having the means for preventing an unactivated lancet from further lateral movement (20) located much closer to the lancet disposition aperture (8) than that of the preferred embodiment. Both the placement of a fresh lancet (26) and the activation of the fresh lancet is as described for the preferred embodiment. The removal of the used lancet (27) is effected by inserting a fresh lancet (26) through the lancet insertion aperture (9) until the slider (22) of the fresh lancet (26) comes into contact with the means for preventing lancet from further lateral movement (20) and is positioned in place. As the fresh lancet (26) is positioned in place, it pushes the used lancet (27) through the lancet disposition aperture (8) and when the vertical axis of the used lancet is pushed past the lancet disposition aperture (8), it falls out of the activator.

It will be understood that the above descriptions do not limit the invention to the above-given details. It is contemplated that various modifications and substitutions can be made without departing from the spirit and scope of the following claims.

## Claims

1. A lancet activating device for comprising:
a. a housing (3) with a cavity (5,6);
b. at least a first aperture (8, 9) on said housing (3) to allow at least one means of lancet access into said cavity (6);
c. at least a second aperture (7) on said housing (3) to allow at least one means of finger access into said cavity (5);
d. means for positioning a finger (23) within said cavity (5) to receive the activation of a lancet (26);
e. an activating means (4) within said housing (3) to activate a lancet (26);
wherein when a lancet (26) is inserted into said lancet access means and a finger (23) is retrievably inserted into said finger access means and said finger (23) is positioned by said finger-positioning means to receive the activation of a lancet (26), said activating means (4) is activated to trigger said lancet (26) to prick said finger (23).

2. A lancet activating device as claimed in Claim 1 wherein said lancet access means is perpendicular to said finger access means.

3. A lancet activating device as claimed in Claim 1 wherein said activating means (4) comprises of a reciprocating plunger (17).

4. A lancet activating device as claimed in Claims 1 and 3 wherein said activating means (4) is triggered by mechanical, electrical, electrochemical, hydraulic or pneumatic means.

5. A lancet activating device as claimed in Claim 1 wherein said means for positioning a finger comprises at least one leaf-spring (10) mounted onto the housing (3).

6. A lancet activating device as claimed in Claim 1 wherein said means for positioning a finger is detachably mounted on said housing (3).

7. A lancet activating device as claimed in Claims 5 & 6 wherein said means for positioning a finger can comprise at least a rest pad for said finger (23).

8. A lancet activating device as claimed in Claim 1 further comprising a stopper (21) to limit the positioning of said finger (23).

9. A lancet activating device as claimed in Claim 1 wherein said lancet access means is provided with a pair of grooves (19) to guide the access of a lancet (26).

10. A lancet activating device as claimed in Claims 1 and 9 wherein said lancet access means is provided with two apertures; wherein the first aperture (9) is longer than the second aperture (8).

11. A lancet activating device as claimed in Claim 10 wherein the longer aperture (9) is located vertically higher on the housing than the smaller aperture (8).

12. A lancet activating device as claimed in Claims 1 and 11 further comprising at least a means for determining the positioning of a lancet (20).

13. A lancet activating device as claimed in Claim 1 wherein said lancet activating device is detachably attached to a container (2) for receiving and storing at least a used lancet (27).

14. A lancet activating device as claimed in Claim 1 wherein said lancet activating device is permanently attached to a container (2) for receiving and storing at least a used lancet (27).

15. A lancet activating device as claimed in Claims 1 to 14 constructed from any constructible material.

16. A lancet activating device as claimed in Claims 1 to 15 constructed from polymeric materials.

17. A lancet activating device as claimed in Claims 13 and 14 wherein said container (2) is constructed from any constructible material.

18. A lancet activating device as claimed in Claims 13, 14 and 17 wherein said container (2) is constructed from polymeric materials.

19. A lancet activating device as claimed in Claims 13, 14, 17 and 18 wherein the container (2) is provided with an opening (16) on the top of the container to receive used lancets (27).

20. A lancet activating device as claimed in Claim 19 wherein the container (2) is provided with a lid (14) pivotally mounted on the top of the container (2) to close said opening (16).
